# EUROPÄISCHE PATENTSCHRIFT

(11) **EP 2 124 706 B1**
(45) Veröffentlichungstag und Bekanntmachung des Hinweises auf die Patenterteilung: **27.07.2016**
(21) Anmeldenummer: 08715835.8
(22) Anmeldetag: 18.02.2008
(51) Int. Cl.: A61B 1/00, A61B 1/018, A61B 1/227, A61B 1/233, A61B 1/267, A61B 1/12

(54) **SCHLAUCHANORDNUNG FÜR EIN ENDOSKOP**
TUBE ASSEMBLY FOR AN ENDOSCOPE
SYSTÈME TUBULAIRE POUR ENDOSCOPE

(30) Priorität: 19.02.2007 DE 102007008099
(43) Veröffentlichungstag der Anmeldung: 02.12.2009
(73) Patentinhaber: INGOSCOPE SYSTEMS GmbH, 10999 Berlin (DE)
(72) Erfinder: MATTEJA, Bruno, I-10010 Mercenasco (TO) (IT)
(74) Vertreter: Manitz, Finsterwald & Partner GbR
(86) Internationale Anmeldenummer: PCT/EP2008/001238
(87) Internationale Veröffentlichungsnummer: WO 2008/101653

(56) Entgegenhaltungen:
- EP-A- 1 477 104
- WO-A-01/49165
- WO-A-2004/103157
- WO-A-2006/033671
- WO-A1-01/87144
- US-A- 5 702 347
- US-A1- 2003 040 657
- US-A1- 2003 176 766

## Beschreibung

Die Erfindung betrifft das Gebiet der Endoskopie, insbesondere eine Schlauchanordnung zur Befestigung an einem Endoskop.

In vielen Teilbereichen der Medizin, insbesondere in der Gastroenterologie, ist die Endoskopie eine weit verbreitete und erfolgreiche Methode in der Diagnostik und bei der Behandlung einer Vielzahl von Erkrankungen. Dabei hat es sich als zweckmäßig erwiesen, bildgebende Endoskope mit Schlauchanordnungen zu versehen, die einen Kanal oder mehrere Kanäle beispielsweise zur Führung von Arbeitsgeräten aufweisen. Allerdings erweisen sich herkömmliche Schlauchanordnungen der vorgenannten Art häufig als unpraktisch, da die Befestigungsmittel zur Verbindung des Endoskops mit der Schlauchanordnung oft raumgreifend gestaltet sind und/oder die Bedienung des Endoskops behindern. Weiterhin ist die Anordnung der Kanäle häufig sehr spezialisiert konzipiert, so dass für jeden Anwendungszweck eine entsprechende Schlauchanordnung gewählt werden muss.

Aus dem Dokument EP 1 477 104 A ist eine Schlauchanordnung für ein Endoskop gemäß dem Oberbegriff des Anspruchs 1 bekannt.

Das Dokument WO 01/87144 A1 zeigt eine Abschlusskappe zur Befestigung von Zubehör an dem distalen Ende eines Endoskops.

Weitere Schlauchanordnungen für Endoskope sind aus den Dokumenten WO 2004/103157 A, WO2006/033671 A und US-A-5 702 347 bekannt.

Es ist die Aufgabe der Erfindung, eine Schlauchanordnung für ein Endoskop zu schaffen, die eine zuverlässige und platzsparende Befestigung an dem Endoskop ermöglicht. Das Befestigen des Endoskops soll außerdem einfach zu bewerkstelligen sein.

Die Lösung dieser Aufgabe erfolgt durch die Merkmale des Anspruchs 1.

Die erfindungsgemäße Schlauchanordnung für ein Endoskop umfasst zumindest einen Einwegschlauch, der einen Arbeitskanal umgibt. Weiterhin umfasst die Schlauchanordnung Endoskopbefestigungsmittel zum Befestigen des Einwegschlauchs an einem Endoskop. Die Endoskopbefestigungsmittel weisen eine Fixiereinrichtung auf, durch die der Einwegschlauch am distalen Ende des Endoskops drehfest und axial fest fixierbar ist. Die Endoskopbefestigungsmittel weisen ferner wenigstens eine Führungseinrichtung auf, durch die der Einwegschlauch an einem jeweiligen vom distalen Ende des Endoskops beabstandeten Abschnitt des Endoskops verschieblich befestigbar ist.

Mit anderen Worten wird die Verbindung zwischen dem Endoskop und der erfindungsgemäßen Schlauchanordnung durch Endoskopbefestigungsmittel hergestellt, die einerseits eine Fixiereinrichtung, andererseits eine Führungseinrichtung umfassen. Die Fixierungseinrichtung stellt eine feste Verbindung zwischen dem Endoskop und der Schlauchanordnung her, wodurch Relativbewegungen zwischen dem distalen Ende des Endoskops und der Schlauchanordnung unterbunden werden. Hierdurch ist gewährleistet, dass eine in der Umgebung der Fixiereinrichtung angeordnete Austrittsöffnung des Arbeitskanals im Einsatz des Endoskops und insbesondere auch bei einer Krümmung des Endoskops (z. B. aufgrund einer Betätigung eines Bowdenzugs) ihre Position relativ zu dem distalen Ende des Endoskops stets beibehält. Somit ist eine definierte Position einer seitlichen oder axialen Öffnung des Arbeitskanals relativ zu dem distalen Ende des Endoskops sichergestellt, was beispielsweise für das Absaugen von Sekreten oder für eine Probenentnahme mittels einer durch den Arbeitskanal geführten Biopsiezange wichtig ist, wenn zugleich eine Beobachtung mittels des Endoskops erfolgen soll.

Demgegenüber erlaubt die Führungseinrichtung eine Relativbewegung zwischen dem Endoskop und der Schlauchanordnung. Dies ermöglicht Ausgleichsbewegungen in Längsrichtung (längsverschiebliche Befestigung) und/oder in Umfangsrichtung der Schlauchanordnung (drehbewegliche Befestigung), die aufgrund von während der Benutzung auftretenden Biegungen oder Krümmungen entlang der Einheit aus Endoskop und Schlauchanordnung erforderlich sind. Darüber hinaus wird das Befestigen des Endoskops vereinfacht, da eine kraftschlüssige Fixierung in der Regel nur an einem Punkt des Endoskops - nämlich an dessen distalen Ende - erfolgt.

Die Schlauchanordnung umfasst ferner eine Abschlusskappe, welche mit dem distalen Ende des Einwegschlauchs verbunden ist. Die Schlauchanordnung ist somit in axialer Richtung zumindest zweiteilig. Eine solche Abschlusskappe kann kostengünstig hergestellt werden - beispielsweise als Spritzgussteil aus Kunststoff - und eröffnet zusätzliche Möglichkeiten, die Schlauchanordnung an die jeweils vorliegenden Bedürfnisse anzupassen.

Die Abschlusskappe ist fest mit der Fixiereinrichtung verbunden. Wie bereits vorstehend erläutert, besteht zwischen der Fixiereinrichtung und dem Endoskop eine drehfeste und axial feste Verbindung. Ist nun die Fixereinrichtung fest mit der Abschlusskappe verbunden, ist besonders zuverlässig sichergestellt, dass die Abschlusskappe bei endoskopischen Untersuchungen und/oder Behandlungen nicht verloren geht. Außerdem ist dadurch die relative Lage der Abschlusskappe bezüglich der Optik des Endoskops fest definiert, was bei verschiedenen Untersuchungs-/Behandlungsmethoden vorteilhaft sein kann.

Gemäß einer vorteilhaften Weiterbildung umgibt der Einwegschlauch zusätzlich wenigstens einen Spülkanal, wobei die Schlauchanordnung eine Austrittsöffnung des Spülkanals aufweist, die bezüglich der Fixiereinrichtung für das distale Ende des Endoskops nach distal versetzt angeordnet ist. Die Schlauchanordnung weist ferner eine Öffnung des Arbeitskanals auf, die bezüglich der Fixiereinrichtung ebenfalls nach distal versetzt angeordnet ist. Somit ist beispielsweise sichergestellt, dass eine am distalen Ende des Endoskops befindliche Optik mittels einer durch den Spülkanal geführten Spülflüssigkeit stets zuverlässig gespült werden kann. Die Schlauchanordnung umgibt in diesem Fall also zumindest zwei Kanäle, einen Arbeitskanal und einen Spülkanal. Die Austrittsöffnung des Spülkanals und die Öffnung des Arbeitskanals sind in einem Abschnitt der Schlauchanordnung angeordnet, der in distaler Richtung der Schlauchanordnung über die Fixiereinrichtung für das distale Endes des Endoskops hinaus ragt, wodurch ein effizientes Spülen einer am distalen Ende des Endoskops angeordneten Optik mittels einer aus der Austrittsöffnung des Spülkanals austretenden Spülflüssigkeit möglich ist. Außerdem können mittels des Arbeitskanals durchgeführte Tätigkeiten visuell durch das Endoskop kontrolliert werden.

Gemäß einer Ausführungsform der erfindungsgemäßen Schlauchanordnung umfasst diese einen einzigen Einwegschlauch, an dem der Arbeitskanal und gegebenenfalls der Spülkanal ausgebildet sind.

Es ist bevorzugt, wenn die Abschlusskappe einstückig mit der Fixiereinrichtung ausgebildet ist.

Bei einer Weiterbildung der Abschlusskappe sind die Austrittsöffnung des Spülkanals und/oder die Öffnung des Arbeitskanals an der Abschlusskappe ausgebildet.

Es kann bei vorteilhaften Ausführungsformen der erfindungsgemäßen Schlauchanordnung mit Abschlusskappe vorgesehen sein, dass die Öffnung des Arbeitskanals und/oder die Austrittsöffnung des gegebenenfalls vorhandenen Spülkanals seitlich angeordnet sind, wodurch beispielsweise ein Spülen der Endoskopoptik mittels über den Spülkanal zugeführter Spülflüssigkeit ermöglicht wird. Unter anderem für diesen Zweck ist der Spülkanal - bezogen auf einen Querschnitt der Schlauchanordnung - vorzugsweise zwischen dem Arbeitskanal und den Endoskopbefestigungsmitteln angeordnet.

Gemäß einer Ausführungsform ist der gegebenenfalls vorhandene Spülkanal in einem Querschnitt der Schlauchanordnung - bezogen auf eine Verbindungslinie zwischen einer Längsachse des Einwegschlauchs und einer Längsachse der Endoskopbefestigungsmittel - seitlich versetzt angeordnet. Anstelle eines einzigen Spülkanals kann die Schlauchanordnung zwei oder mehr Spülkanäle aufweisen. Die Austrittöffnungen der Spülkanäle können in axialer Richtung der Schlauchanordnung versetzt zueinander angeordnet sein. Ein relativer Versatz der Austrittöffnungen in radialer Richtung oder in Umfangsrichtung kann zusätzlich oder alternativ vorgesehen sein. Insbesondere sind die Spülkanäle - bezogen auf die vorstehend genannte Verbindungslinie - symmetrisch angeordnet.

Gemäß einer Ausführungsform der erfindungsgemäßen Schlauchanordnung umfasst die Führungseinrichtung mehrere Halteelemente, die beabstandet voneinander entlang der Länge des Einwegschlauchs angeordnet sind, da eine durchgehende, d.h. unterbrechungsfreie, Führungseinrichtung ist in vielen Fällen nicht notwendig. Eine mehrere Halteelemente umfassende Führungseinrichtung erlaubt zudem ein leichteres Einführen des Endoskops in die Führungseinrichtung und bedeutet bei einer Relativbewegung von Endoskop und Einwegschlauch im Betrieb geringe zu überwindende Reibungskräfte. Insbesondere können zwei bis drei im Wesentlichen identische Halteelemente vorgesehen sein, beispielsweise bei einer Endoskoplänge von 35 cm. Bei einer Endoskoplänge von 75 cm können beispielsweise drei bis fünf Halteelemente vorgesehen sein, bei einer Endoskoplänge von 100 cm beispielsweise fünf bis sieben Halteelemente.

Es ist bevorzugt, wenn die Führungseinrichtung wenigstens eine im Wesentlichen hohlzylinderförmig ausgebildete Schlaufe aufweist. Derartige Schlaufen sind leicht zu fertigen bzw. anzuformen, stellen gleichzeitig aber auch eine zuverlässige Führung der Schlauchanordnung sicher.

Die Führungseinrichtung und/oder die Fixiereinrichtung können als einstückiges Befestigungselement ausgebildet sein, das den Einwegschlauch und das Endoskop umgibt, und das eine Einwegschlauchaufnahme und eine Endoskopaufnahme aufweist, die durch eine Einschnürung und/oder einen Steg definiert sind, die bzw. der zwischen der Einwegschlauchaufnahme und der Endoskopaufnahme angeordnet ist.

Die Fixiereinrichtung kann derart gestaltet sein, dass eine kraftschlüssige, insbesondere reibschlüssige Verbindung zwischen dem Einwegschlauch und dem Endoskop hergestellt wird. Eine vorteilhafte Ausgestaltung der Fixiereinrichtung umfasst eine in axialer Richtung beidseitig geöffnete Manschette.

Gemäß einer Ausführungsform der Schlauchanordnung weist der Einwegschlauch zumindest ein Verstärkungselement auf. Das Verstärkungselement ist krümmbar, da es in erster Linie zur Stabilisierung des Querschnitts der Kanäle dient, die beispielsweise bei einer starken Krümmung des Einwegschlauchs zusammengequetscht werden würden, und weniger zur Stabilisierung der Längsachse der Schlauchanordnung, obwohl dieser Aspekt in speziell gelagerten Fällen auch genutzt werden kann. Die Steifigkeit des Querschnitts des Verstärkungselements und die Elastizität gegenüber Kräften senkrecht zu der Längsachse des Verstärkungselements kann entsprechend den Anforderungen gewählt werden.

Das Verstärkungselement ist insbesondere in einem distalen Bereich der Schlauchanordnung angeordnet, der relativ zu der Austrittsöffnung des gegebenenfalls vorhandenen Spülkanals nach proximal versetzt angeordnet ist, da hier bei einer entsprechender Betätigung des Endoskops die stärksten Krümmungen zu erwarten sind. Es können aber auch zusätzliche Verstärkungselemente in weiteren kritischen Bereichen der Schlauchanordnung vorgesehen sein.

Die Erfindung wird im Folgenden rein beispielhaft anhand vorteilhafter Ausführungsformen unter Bezugnahme auf die Zeichnungen beschrieben. Es zeigen:
- Fig. 1a: eine schematische Darstellung einer Schlauchanordnung (keine Ausführungsform der Erfindung);
- Fig. 1b: eine um 90° gedrehte Ansicht der Schlauchanordnung von Fig. 1a;
- Fig. 1c: einen Schnitt durch die Schlauchanordnung von Fig. 1a entlang einer Schnittlinie AA;
- Fig. 1d: einen Schnitt durch eine Fixiermanschette der Schlauchanordnung entlang der Schnittlinie BB in Fig. 1b;
- Fig. 2a: eine schematische Darstellung einer weiteren Schlauchanordnung (keine Ausführungsform der Erfindung);
- Fig. 2b: eine um 90° gedrehte Ansicht der Schlauchanordnung von Fig. 2a;
- Fig. 2c: eine schematische Darstellung einer Ausführungsform eines Innenschlauchs;
- Fig. 2d: eine vergrößerte Ansicht des distalen Endes des Innenschlauchs von Fig. 2c;
- Fig. 3a: eine schematische Darstellung einer Ausführungsform der erfindungemäßen Schlauchanordnung;
- Fig. 3b: eine schematische Schnittansicht einer Ausführungsform einer Abschlusskappe mit Fixiereinrichtung;
- Fig. 3c: eine perspektivische Ansicht der Abschlusskappe von Fig. 3b;
- Fig. 4a: eine schematische Schnittansicht einer weiteren Ausführungsform einer Abschlusskappe mit Fixiereinrichtung;
- Fig. 4b: eine perspektivische Ansicht der Abschlusskappe von Fig. 4a;
- Fig. 5a: eine schematische Darstellung einer Schlauchanordnung mit Verstärkungselement (keine Ausführungsform der Erfindung);
- Fig. 5b: einen Schnitt durch die Schlauchanordnung von Fig. 5a entlang einer Schnittlinie CC;
- Fig. 5c: ein Endoskop mit einer Schlauchanordnung in einer gekrümmten Anordnung,
- Fig. 6a und 6b: jeweils einen Längsschnitt einer Schlauchanordnung mit einem Abschlussstück (keine Ausführungsform der Erfindung),
- Fig. 7a und 7b: jeweils einen Querschnitt einer Schlauchanordnung mit zwei versetzt und symmetrisch angeordneten Spülkanälen bzw. mit einem versetzt angeordneten Spülkanal,
- Fig. 8a bis 8c: verschiedene Ausführungsformen der Fixiereinrichtung und/oder der Führungseinrichtung in einer jeweiligen Querschnittsansicht.

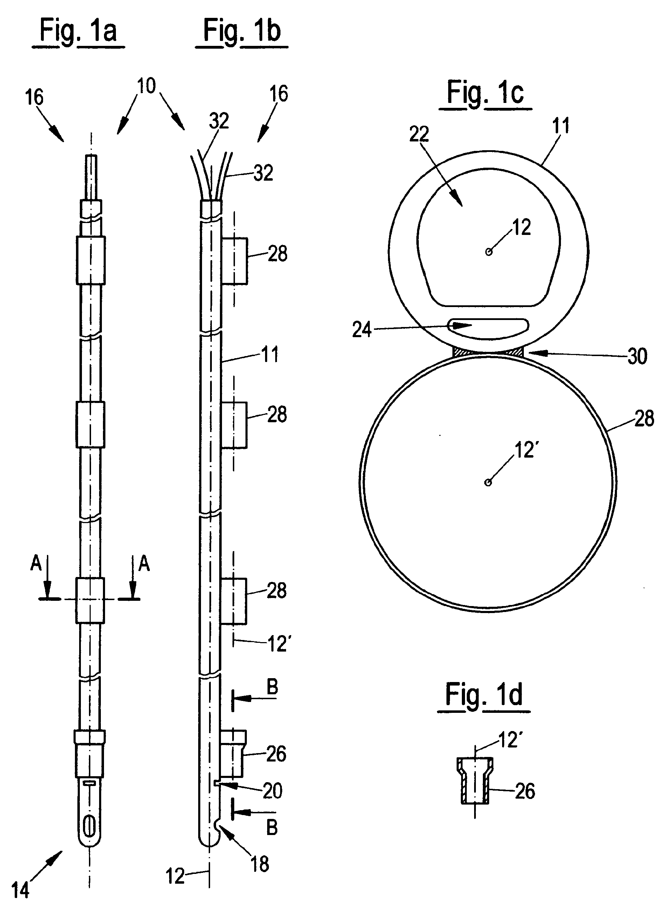

Fig. 1a und 1b (keine Ausführungsform der Erfindung) zeigen eine Schlauchanordnung 10 mit einem flexiblen Einwegschlauch 11, wobei die Fig. 1b eine gegenüber der Fig. 1a um 90° um eine Längsachse 12 verdrehte Ansicht darstellt. Die Schlauchanordnung 10 weist ein distales Ende 14 und ein proximales Ende 16 auf. In einem distalen Bereich der Schlauchanordnung 10 nahe dem distalen Ende 14 ist eine Fixiermanschette 26 an dem Einwegschlauch 11 befestigt. Die Fixiermanschette 26 ist weiter proximal angeordnet als eine Öffnung 18 und eine Austrittsöffnung 20, so dass das distale Ende eines in die Fixiermanschette 26 einzuführenden Endoskops (nicht gezeigt) in visuellem Kontakt mit den - bezüglich der Längsachse 12 - seitlich an dem Einwegschlauch 11 angebrachten Öffnungen 18, 20 steht. Die Öffnung 18 und die Austrittsöffnung 20 stehen mit einem Arbeitskanal 22 bzw. einem optionalen Spülkanal 24 (gezeigt in Fig. 1c) des Einwegschlauchs 11 in Verbindung. Wie den Fig. 1a und 1b deutlich zu entnehmen ist, ist die Austrittsöffnung 20 des Spülkanals 24 in geringem Abstand nach distal versetzt zu der Fixiermanschette 26 angeordnet. Durch die räumliche Nähe und die schlitzartige Formgebung der somit als Düse wirkenden Austrittsöffnung 20 kann Spülflüssigkeit auf die Optik des Endoskops gesprüht werden, um diese zu reinigen und beispielsweise störendes Sekret zu entfernen. Alternativ hierzu kann auch Luft durch den Spülkanal 24 geführt werden, um nicht nur die Optik des Endoskops freizublasen, sondern um durch Luftinsuflation auch den zu untersuchenden Hohlraum aufzublasen, so dass eine Wand beispielsweise des Magens oder des Ösophagus besser beobachtet werden kann.

Die Öffnung 18 des Arbeitskanals 22 ist relativ zu der Fixiermanschette 26 weiter nach distal versetzt als die Austrittsöffnung 20. Da auch sie bezüglich der Längsachse 12 des Einwegschlauchs 11 auf der der Fixiermanschette 26 zugewandten Seite angeordnet ist, ist es beispielsweise möglich, Sekret oder andere Substanzen im Bereich direkt vor dem Endoskop abzusaugen.

Neben der Fixiermanschette 26 sind Führungsschlaufen 28 an dem Einwegschlauch 11 der Schlauchanordnung 10 angebracht. Sie sind in regelmäßigen Abständen angeordnet, wobei dies lediglich eine spezielle Ausführungsform darstellt.

Fig. 1c verdeutlicht den Aufbau der Schlauchanordnung anhand eines Querschnitts entlang der Schnittlinie AA von Fig. 1a. Im oberen Teil der Fig. 1c ist der Querschnitt des Einwegschlauchs 11 zu sehen. Der Einwegschlauch 11 umfasst - wie vorstehend bereits angesprochen - den Arbeitskanal 22 und den optionalen Spülkanal 24. Zur besseren Nutzung der Querschnittsfläche des Einwegschlauchs 11 sind weder der Arbeitskanal 22 noch der Spülkanal 24 kreisförmig ausgebildet. In der dargestellten Ausführungsform der Schlauchanordnung 10 weist der Arbeitskanal 22 eine wesentlich größere Querschnittsfläche auf als der Spülkanal 24. Je nach Bedarf kann eine davon abweichende Aufteilung gewählt werden. Der Querschnitt des Arbeitskanals 22 lässt sich vereinfacht durch eine Kombination eines Kreisbogenabschnitts mit einem Teil eines Trapezes mit abgerundeten Ecken beschreiben. Die Abweichung von einem kreisförmigen Querschnitt des Arbeitskanals 22 bei gegebenem Außendurchmesser des Einwegschlauchs 11 erlaubt eine effizientere Durchführung von Flüssigkeit oder Gewebestücken. Außerdem lässt sich ein Arbeitsgerät mit kreisförmigem Querschnitt in einem derartig geformten Arbeitskanal 22 leichter bewegen, da die Kontaktfläche zu den Innenwänden des Arbeitskanals 22 kleiner ist als im Fall eines kreisförmigen Querschnitts und somit weniger Reibung auftritt.

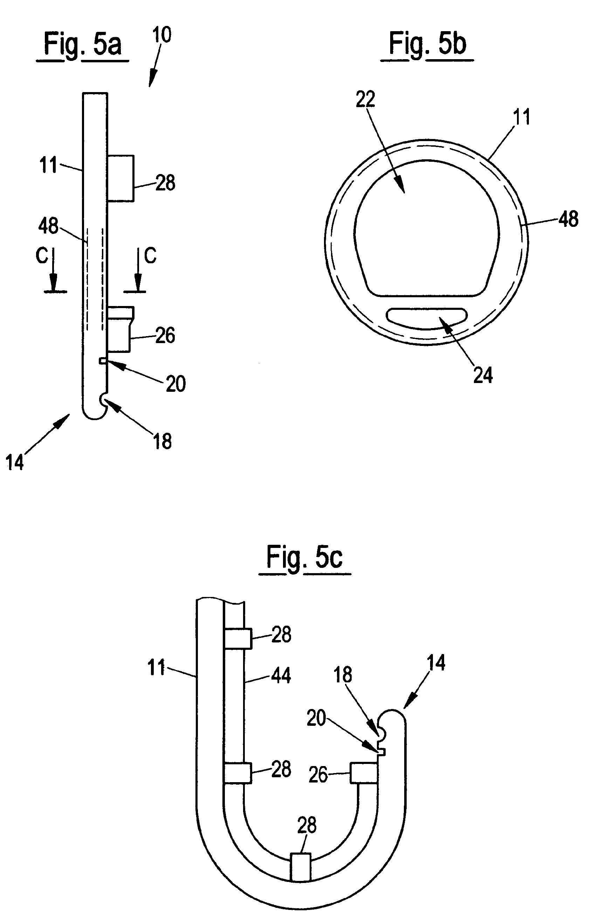

Insbesondere sind die den Arbeitskanal 22 seitlich begrenzenden Seitenwände des Einwegschlauchs beispielsweise bezüglich des dem Spülkanal 24 gegenüberliegenden Wandabschnitts des Einwegschlauchs 11 relativ dick ausgebildet. Hierdurch wird bei einem Krümmen des Einwegschlauchs 11 mittels des Endoskops (vgl. Fig. 5c) ein Kollabieren des Arbeitskanals 22 verhindert.

Der Spülkanal 24 ist in seiner Querschnittsform und -größe derart optimiert, dass stets eine ausreichende Zufuhr von Spülflüssigkeit zur Reinigung der Optik des Endoskops bei optimaler Querschnittsflächenausnutzung des Einwegschlauchs 11 geliefert werden kann. Der Spülkanal 24 liegt zwischen dem Arbeitskanal 22 und den Führungsschlaufen 28, d. h. auf einer Linie, die die Längsachse 12 des Einwegschlauchs 11 und eine parallel dazu verlaufende Längsachse 12' verbindet, die sich entlang der Längsachsen der Führungsschlaufen 28 und der Fixiermanschette 26 erstreckt.

Der untere Teil des Bildes in Fig. 1c wird von dem Querschnitt einer der Führungsschlaufen 28 eingenommen. Diese weist einen kreisförmigen Querschnitt auf, der zur Aufnahme des Endoskops dient. Innerhalb der Führungsschlaufe 28 kann sich das Endoskop insbesondere in Längsrichtung bewegen. Die Führungsschlaufen 28 dienen somit dazu, den Einwegschlauch 11 der Schlauchanordnung 10 in seitlicher Richtung in enger räumlicher Nähe zu dem Endoskop zu halten, sie allerdings nicht fest miteinander zu verbinden. Dies ist, wie vorstehend bereits erläutert, Aufgabe der Fixiermanschette 26. Die Führungsschlaufe 28 ist an den Einwegschlauch angeklebt, wie durch die Klebeverbindung 30 angedeutet ist. Die Art der Verbindung kann allerdings frei gewählt werden, beispielsweise ist alternativ eine Schweißverbindung oder eine Schrumpfverbindung möglich. Auch eine einstückige Ausführung des Einwegschlauchs 11 und der Führungsschlaufen 28 ist denkbar. Entsprechendes gilt für die Fixiermanschette 26.

Fig. 1d zeigt einen Querschnitt durch die Fixiermanschette 26 entlang der Schnittlinie BB von Fig. 1b. Es ist deutlich zu erkennen, dass der Innendurchmesser der Fixiermanschette 28 distal kleiner ist als proximal. Vorzugsweise besteht die Fixiermanschette 26 aus einem elastischem Material, so dass durch das Einführen des distalen Endes des Endoskops der innenradiusreduzierte Bereich der Fixiermanschette 26 geweitet wird. Dadurch entsteht eine reibschlüssige Verbindung zwischen der Fixiermanschette 26 - die ja fest mit dem Einwegschlauch 12 verbunden ist - und dem Endoskop. Eine zuverlässige Fixierung ist somit sichergestellt.

Am proximalen Ende des Einwegschlauchs 11 sind der Arbeitskanal 22 und der Spülkanal 24 mit separaten Verbindungsschläuchen 32 verbunden, die zur Zu-/Abfuhr von Flüssigkeiten und/oder Gewebeteilen dienen.

Der Spülkanal 24 kann bei der Ausführungsform gemäß Fig. 1a bis 1d auch entfallen, insbesondere für Anwendungen der Schlauchanordnung 10 im Hals-Nasen-Ohren-Bereich (HNO). In diesem Fall entfällt die in Fig. 1a und 1b gezeigte Austrittsöffnung 20.

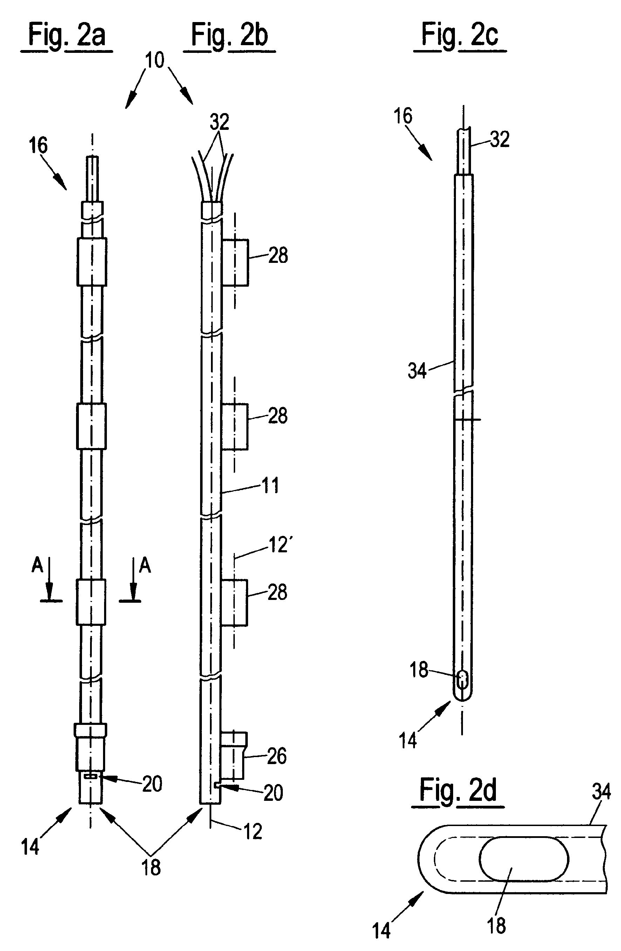

Eine etwas abgewandelte Ausführungsform der Schlauchanordnung 10 ist in Fig. 2a und 2b gezeigt (keine Ausführungsform der Erfindung). In vielen Aspekten ähneln sich die beiden Ausführungsformen, insbesondere hinsichtlich der Querschnittsform der Schlauchanordnung 10. Im distalen Bereich liegt eine Übereinstimmung hinsichtlich der Fixiermanschette 26 und der Austrittsöffnung 20 vor. Die Öffnung 18 des Arbeitskanals 22 ist allerdings nicht seitlich angebracht, sondern ist bezüglich der Längsachse 12 axial ausgerichtet. Mit anderen Worten ist das distale Ende des Einwegschlauchs 11 senkrecht zur Längsachse 12 abgeschnitten. Einerseits kann auch diese Ausführungsform in der vorliegenden Form beispielsweise zum Absaugen von Substanzen verwendet werden. Andererseits eignet sich diese Ausführungsform aber insbesondere zum Führen von Arbeitsgerätschaften durch den Arbeitskanal 22. Dies gilt insbesondere, wenn der Spülkanal 24 mit der Austrittsöffnung 20 entfällt. Optional kann der Arbeitskanal 22 aber auch ähnlich der in Fig. 1a und b gezeigten Ausführungsform genutzt werden, wenn ein Innenschlauch 34 verwendet wird, der durch den Arbeitskanal 22 geführt wird.

Ein derartiger Innenschlauch 34 ist in Fig. 2c gezeigt. An seinem proximalen Ende 16 ist er mit einem Verbindungsschlauch 32 verbunden. Das distale Ende 16 des Innenschlauchs 34 ähnelt der Spitze der in Fig. 1a und 1b dargestellten Ausführungsform der Schlauchanordnung 10. So verfügt der Innenschlauch 34 über ein seitlich angeordnetes Langloch, welches die Öffnung 18 darstellt. Die Öffnung 18 des Innenschlauchs 34 ist detaillierter in Fig. 2d dargestellt, wobei die gestrichelte Linie die Wandstärke des Innenschlauchs 34 symbolisiert. Die Öffnung 18 ist derart dimensioniert, dass sie in einer Richtung senkrecht zur Längsachse 12 größtmögliche Breite aufweist, so dass auch relativ große Gewebestücke bzw. Sekretklumpen abgesaugt werden können.

Der Vorteil dieser Ausführungsform in Kombination mit der Verwendung des Innenschlauchs 34 liegt darin, dass die Öffnung 18 durch Drehen und Hin- und Herschieben des Innenschlauchs 34 fast beliebig relativ zu der Fixiermanschette 26 - und damit relativ zu dem Endoskop - positioniert werden kann. Beispielsweise kann so bei unveränderter Lage des Einwegschlauchs 11 bzw. des Endoskops ein größerer Bereich des Untersuchungsgebiets mit der Öffnung 18 erreicht werden. Dem behandelnden Arzt wird so ein flexibles Arbeitsgerät an die Hand gegeben.

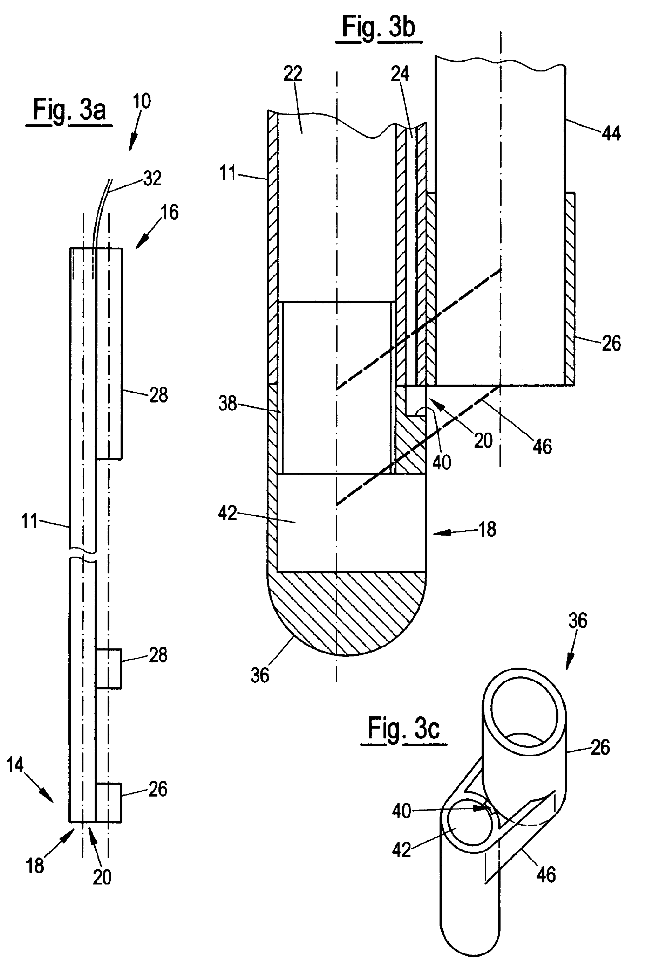

Eine weitere Ausführungsform der Schlauchanordnung 10 ist in Fig. 3a gezeigt. Sie zeigt einen Einwegschlauch 11, der mit Führungsschlaufen 28 und einer Fixiermanschette 26 versehen ist. Die proximale Führungsschlaufe 28 weist eine deutlich größere Längserstreckung auf als die distale Führungsschlaufe 28 der vorstehend behandelten Ausführungsformen. Ein weiterer Unterschied zu den in den Fig. 1a und 1b und 2a und 2b gezeigten Ausführungsformen liegt darin, dass das distale Ende des Einwegschlauchs 11 bündig mit dem distalen Ende der Fixiermanschette 26 abschließt. In das distale Ende des Einwegschlauchs 11, der in diesem Fall einen Arbeitskanal 22 und optional einen Spülkanal 24 umfasst, kann eine Abschlusskappe 36 (z.B. Fig. 3b) eingeführt werden. Es können auch Ausführungsformen vorgesehen sein, bei denen die Abschlusskappe 36 auf den Einwegschlauch 11 aufgesteckt oder anderweitig mit diesem verbunden wird.

Eine Schnittansicht durch das distale Ende 14 der Schlauchanordnung 10 ist in Fig. 3b gezeigt. Der Einwegschlauch 11 weist einen Arbeitskanal 22 und einen Spülkanal 24 auf. In den Arbeitskanal 22 ist eine Verbindungshülse 38 eingesteckt, auf die wiederum die Abschlusskappe 36 aufgesteckt ist. Die Verbindungshülse 38 und die Abschlusskappe 36 können auch einstückig ausgeführt sein.

Die Abschlusskappe 36 weist eine Nut 40 auf. Ist die Abschlusskappe 36 mit dem Einwegschlauch 11 verbunden, bildet diese Nut 40 die Austrittsöffnung 20 des Spülkanals 24. Weiterhin weist die Abschlusskappe 36 einen Kanal 42 auf, der mit dem Arbeitskanal 22 in Verbindung steht und der eine seitlich angeordnete Öffnung 18 umfasst. Funktionell und bezüglich der Anordnung der Öffnungen 18, 20 der Kanäle 22, 24 ähnelt daher die in Fig. 3b dargestellte Ausführungsform der Schlauchanordnung 10 der in Fig. 1a und 1b dargestellten Ausführungsform.

Ein integraler Bestandteil der Abschlusskappe 36 ist weiterhin die Fixiermanschette 26, die ein Endoskop 44 drehfest und axial fest mit der Abschlusskappe 36 und damit dem Einwegschlauch 11 verbindet.

Die detaillierte Darstellung des distalen Endes 14 der Schlauchanordnung 10 verdeutlicht die räumliche Nähe der Öffnungen 18, 20 zu dem distalen Ende des Endoskops 44. Durch die Austrittsöffnung 20 kann effizient Spülflüssigkeit auf die Endoskopoptik gespritzt werden, um diese zu reinigen. Im Gesichtsfeld der Optik des Endoskops kann hingegen durch die Öffnung 18 Sekret oder dergleichen einfach abgesaugt werden.

Die Abschlusskappe 36 ist ein einfach herzustellendes Bauteil und kann beispielsweise verschiedene Variationen der Formen und Orientierungen der Öffnungen 18, 20 aufweisen. Auch können die Öffnungen 18, 20 an unterschiedlichen Seiten angeordnet sein. Der Innenschlauch 34 kann ebenfalls mit einer solchen Abschlusskappe versehen sein, sowohl bei der Ausführungsform des Innenschlauchs 34 mit lediglich einem Arbeitskanal 22 als auch bei der Ausführungsform des Innenschlauchs 34 mit einem Arbeitskanal 22 und einem Spülkanal 24.

Eine perspektivische Ansicht einer solchen Abschlusskappe ist in Fig. 3c gezeigt. Die Abschlusskappe 36 der Fig. 3c verfügt über zwei Verbindungsstege 46, die die Fixiermanschette 26 mit dem die Öffnungen 18, 20 enthaltenden Teil der Abschlusskappe 36 verbindet. Die Lage der Verbindungsstege 46 relativ zu den anderen Elementen der Abschlusskappe 36 ist in Fig. 3b durch schräg zur den Längsachsen 12, 12' verlaufende gestrichelte Linien angedeutet.

Auch bei der Ausführungsform gemäß Fig. 3a bis 3c kann der Spülkanal 24 mit der Austrittsöffnung 20 bzw. 40 entfallen.

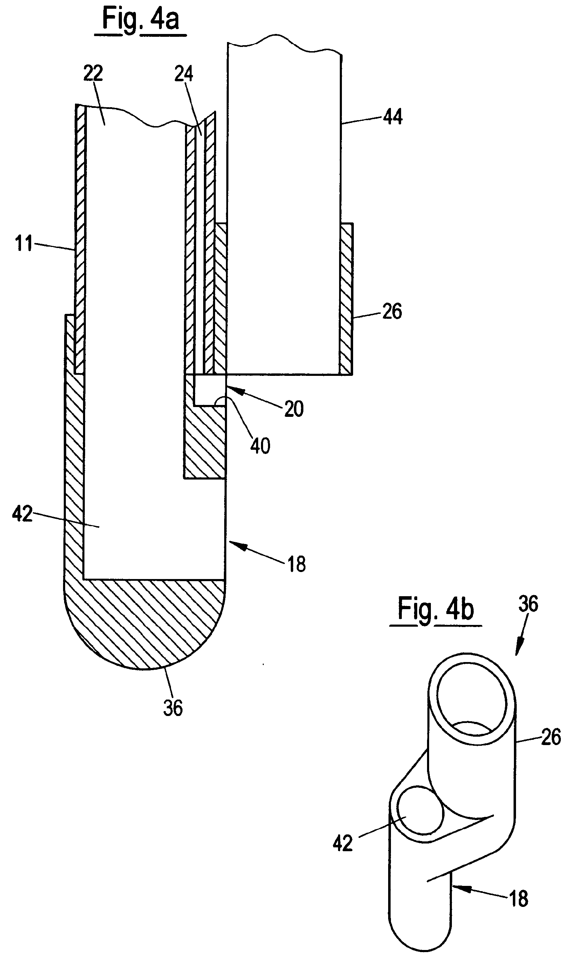

Eine etwas andere Ausführungsform einer Abschlusskappe 36 illustriert Fig. 4a. Bei dieser Ausführungsform wird die Abschlusskappe 36 auf den Einwegschlauch 11 aufgesteckt, so dass auf eine Verbindungshülse 38 verzichtet werden kann. Der übrige Aufbau der Abschlusskappe 36 ähnelt dem der in Fig. 3b gezeigten Ausführungsform.

Fig. 4b zeigt eine perspektivische Ansicht der in Fig. 4a dargestellten Ausführungsform der Abschlusskappe 36. Die die Austrittsöffnung 20 des Spülkanals 24 bildende Nut 40 ist wiederum an dem distalen Ende der Fixiermanschette 26 angeordnet, so dass die Nut 40 in der gezeigten Perspektive gemäß Fig. 4b nicht zu sehen ist.

Eine vorteilhafte Modifikation der Schlauchanordnung 10 ist in Fig. 5a gezeigt (keine Ausführungsform der Erfindung). Die wesentlichen Merkmale des distalen Endes 14 der Schlauchanordnung 10, wie die Öffnung 18, die Öffnung 20 des optionalen Spülkanals, die Fixiermanschette 26 und die Führungsschlaufe 28, sind bereits vorstehend ausführlich beschrieben worden. Die dargestellte Ausführungsform weist allerdings zusätzlich eine Spiralfeder 48 auf, die in den Einwegschlauch 11 eingebettet ist, wie Fig. 5b zu entnehmen ist. Fig. 5b zeigt einen Schnitt senkrecht zu einer Schnittlinie CC von Fig. 5a.

Die Spiralfeder 48 erstreckt sich über einen Abschnitt des distalen Bereichs der Schlauchanordnung 10, der relativ zu der Austrittsöffnung 20 des Spülkanals 24 nach proximal versetzt ist. Sie ist krümmbar, das heißt senkrecht zur Längsachse 12 elastisch biegbar, wobei sich ihre Querschnittsform bei einer Biegung nur unwesentlich ändert. Dadurch wird erreicht, dass bei einer Krümmung der Schlauchanordnung 10 die Kanäle 22, 24 nicht zusammengedrückt werden, wodurch beispielsweise die Ab-/Zufuhr von Flüssigkeit und/oder Gewebe unterbunden werden würde.

Ein derartiges Verstärkungselement in Form einer Spiralfeder 48 - wobei auch andere Verstärkungselemente verwendet werden können - ist besonders in Situationen vorteilhaft, wenn das Endoskop in Inversion gebracht wird. In diesem Zustand wird das Endoskop 44 stark gekrümmt, so dass die Endoskopoptik "rückwärts" nach proximal blickt (siehe Fig. 5c). Ohne die Spiralfeder 48 würde der Schlauchquerschnitt des Einwegschlauchs 11 zusammengequetscht werden. Die Zufuhr beispielsweise von Spülflüssigkeit wäre damit unterbrochen. Die gleiche stützende Wirkung kann beispielsweise durch eine den Einwegschlauch 11 abschnittsweise umgebende Spiralfeder erzielt werden.

Anhand von Fig. 5c wird außerdem deutlich, dass die Führungsschlaufen 28 eine Relativbewegung zwischen dem Endoskop 44 und dem Einwegschlauch 11 zulassen sollen. Wäre die Verbindung zwischen den Führungsschlaufen 28 und dem Endoskop 44 fest und würde keine längsverschiebliche Bewegung zugelassen, so würden aufgrund der unterschiedlichen Krümmungsradien des Einwegschlauchs 11 und des Endoskops 44 starke Dehnbelastungen in Längsrichtung in dem Einwegschlauch 11 oder in dem Endoskop 44 auftreten, die einerseits den Einwegschlauch 11 schädigen könnten, andererseits auch ein Kollabieren der Querschnitte der Kanäle 22, 24 verursachen könnten, oder die eine Beschädigung der Bowdenzüge des Endoskops 44 verursachen könnten.

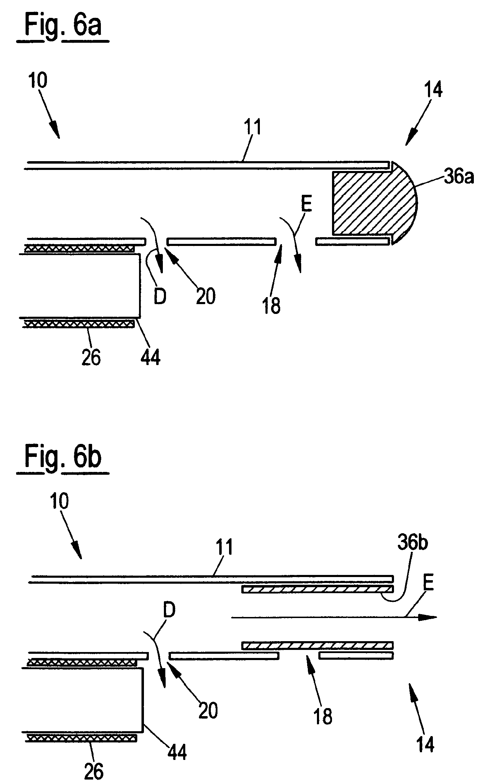

Fig. 6a zeigt das distale Ende 14 einer Ausführungsform der Schlauchanordnung 10 (keine Ausführungsform der Erfindung). Der Arbeitskanal 22 und der optionale Spülkanal 24 des Einwegschlauchs 11 sind nicht gezeigt, da deren Anordnung für den nachfolgend zu beschreibenden Aspekt von untergeordneter Bedeutung ist. Die Pfeile D, E deuten an, dass sowohl der Spülkanal 24 als auch der Arbeitskanal 22 durch die seitlichen Öffnungen 20 bzw. 18 des Einwegschlauchs 11 mit einem Bereich vor dem Endoskop 44 in Verbindung stehen. Das distale Ende 14 der Schlauchanordnung 10 ist durch ein Abschlussstück 36a verschlossen. Die beiden in Längsrichtung des Einwegschlauchs 11 zueinander versetzten Öffnungen 18, 20 sind durch das Abschlussstück 36a jedoch nicht verschlossen.

Fig. 6b zeigt eine andere Ausführungsform der Schlauchanordnung 10 (keine Ausführungsform der Erfindung), die allerdings auf den gleichen Einwegschlauch 11 zurückgreift, wie die in Fig. 6a gezeigte Ausführungsform. Das kappen- oder stöpselartige Abschlussstück 36a der Fig. 6a wurde hier durch ein hohlzylinderförmiges Abschlussstück 36b ersetzt, welches die axiale Öffnung am distalen Ende 14 des Einwegschlauchs 11 offen lässt. Das Abschlussstück 36b verschließt die seitliche Arbeitskanalöffnung 18, so dass der Arbeitskanal 22 nun mit einem Bereich vor dem distalen Ende 14 der Schlauchanordnung 10 in Verbindung steht. Die gegebenenfalls vorhandene seitliche Austrittsöffnung 20 des Spülkanals 24 ist weiterhin geöffnet.

Dies zeigt exemplarisch, dass durch die Gestaltung des Abschlussstücks 36a, 36b verschiedene Schlauchanordnungskonfigurationen realisiert werden können, ohne dass eine andere Ausführungsform des Einwegschlauchs 11 verwendet werden muss. Dies ermöglicht eine kostengünstige Herstellung der beiden unterschiedlichen Konfigurationen (Einwegschlauch 11 als Gleichteil) und ermöglicht auch die Verwendung von kostengünstig herzustellender Endlosware für einen Einwegschlauch 11.

Abweichend von Fig. 6a und 6b können die Abschlussstücke 36a, 36b auch so gestaltet sein, dass sie auf den Einwegschlauch 11 aufgesteckt werden - nicht eingesteckt wie in Fig. 6a und 6b - oder auf andere Weise an diesem befestigt werden. Entsprechende Abschlussstücke 36a, 36b können auch bei Ausführungsformen der Schlauchanordnung 10 mit Innenschlauch 34 Verwendung finden.

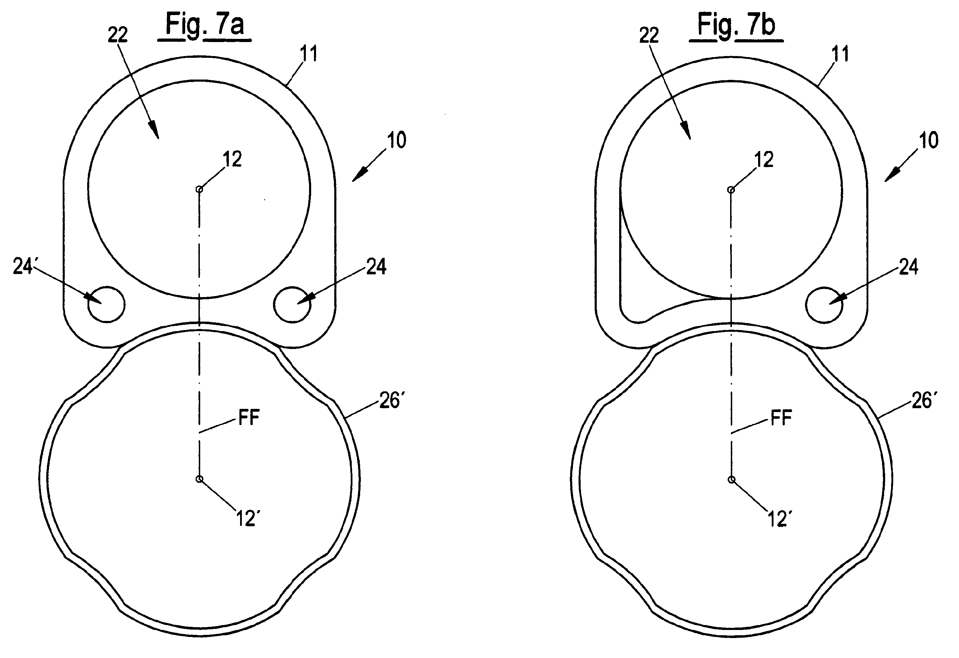

Die erfindungsgemäße Schlauchanordnung 10 kann mehr als einen Spülkanal 24 aufweisen, wie in Fig. 7a gezeigt ist. Diese Ausführungsform weist zwei Spülkanäle 24, 24' auf, die nicht direkt zwischen dem Arbeitskanal 22 und der Fixiereinrichtung - hier eine Fixiermanschette 26' mit einem abschnittsweise von einem Kreis abweichenden Querschnitt - angeordnet sind. In einem Querschnitt senkrecht zur Längserstreckung der Schlauchanordnung 10 liegen die Spülkanäle 24, 24' relativ zu einer Verbindungslinie FF zwischen der Längsachse 12 des Einwegschlauchs 11 und der Längsachse 12' der Fixiermanschette 26' seitlich versetzt. Sie sind symmetrisch beidseitig der Verbindungslinie FF angeordnet.

Die versetzte Anordnung der Spülkanäle 24, 24' verringert die Ausdehnung der Schlauchanordnung 10 in einer Richtung parallel zu der Verbindungslinie FF. Außerdem kann der Einwegschlauch 11 bei einer derartigen Ausgestaltung für einen bestimmten Durchmesser des Arbeitskanals 22 (Lumen) eine geringere Wandstärke besitzen.

Die Austrittsöffnungen 20 der Spülkanäle 24, 24' müssen nicht in Längsrichtung des Schlauchanordnung 10 fluchten oder in einer gemeinsamen Querschnittsebene liegen, sondern können beliebig angeordnet sein, um verschiedene Bereiche- eventuell auch unabhängig voneinander - spülen zu können.

Ein Beispiel für eine Ausführungsform mit einem einzigen versetzten Spülkanal 24 ist in Fig. 7b gezeigt. Der Bereich des Spülkanals 24' der Fig. 7a wurde dem Arbeitskanal zugeschlagen. Dieser Bereich kann beispielsweise zur Führung eines weiteren dünnen Schlauchs oder von Arbeitsmitteln/ Instrumenten genutzt werden.

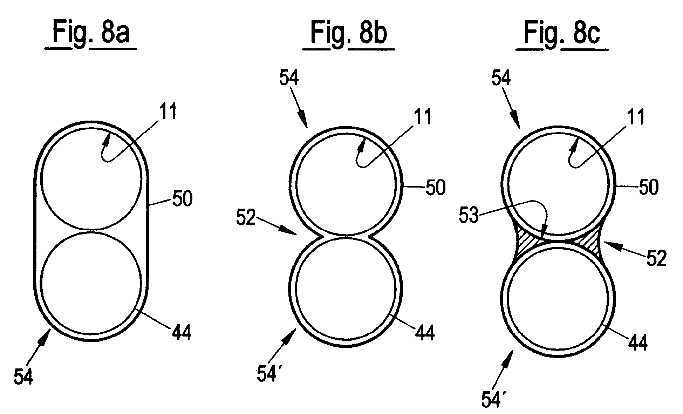

Fig. 8a bis 8c zeigen verschiedene Ausgestaltungen eines Befestigungselements, durch das die Führungsschlaufen 28 und/oder die Fixiermanschette 26, 26' bereitgestellt werden können. Im Wesentlichen wird das Befestigungselement durch ein einfach herzustellendes einstückiges Band 50 gebildet, das sowohl den Einwegschlauch 11 als auch das Endoskop 44 umgibt. Das Band 50 kann lösbar oder fest mit dem Einwegschlauch 11 verbunden sein.

Durch den Begriff "Band" soll nicht suggeriert werden, dass das Band 50 flexibel, beispielsweise als eine Art Gummiband sein muss. Es kann sich dabei beispielsweise auch um ein relativ starres Plastikbauteil handeln.

Wenn das Band 50 als Fixiermanschette 26, 26' fungiert, dann fixiert es das Endoskop 44 drehfest und in axialer Richtung. Dient es lediglich zur Führung des Endoskops 44, so ist es derart ausgebildet, dass sich das Endoskop 44 relativ zu dem Einwegschlauch 11 bewegen kann.

Fig. 8a zeigt eine einfache Variante des Bands 50. Es bildet eine einzige Schlaufe 54, in der der Einwegschlauch 11 und das Endoskop 44 angeordnet sind. Die Schlaufe 54 hat im Wesentlichen die Form einer "O".

Im Gegensatz dazu weist das Band 50 der Fig. 8b eine Taillierung 52 auf, durch die zwei Schlaufen 54, 54' definiert sind, so dass das Band 50 eine Form ähnlich einer unvollkommenen "8" bildet. Mit anderen Worten ist die Einschnürung in mittleren Bereich des Bands 50 nicht vollständig, wodurch eine Verbindung zwischen den beiden Schlaufen 54, 54' besteht. In der Schlaufe 54 ist der Einwegschlauch 11 angeordnet, während die Schlaufe 54' das Endoskop 44 aufnimmt.

Der Vorteil des taillierten Bands 50 besteht darin, dass ein gegenseitiges Verwinden des Einwegschlauchs 11 und des Endoskops 44 verhindert wird. Ein derartiges Verwinden bereitet insbesondere bei einer Inversion des Endoskops 44 Probleme.

Fig. 8c zeigt ein Band 50 mit einer Taillierung 52, dessen Schlaufen 54, 54' durch einen Steg 53 voneinander getrennt sind, wodurch die Form einer "8" gebildet wird. Das Band 50 kann auch lediglich den Steg 53 aufweisen, so dass die Außenkontur des Bands 50 einer "O" entspricht, wie im Fall der in Fig. 8a gezeigten Ausführungsform des Bands 50.

### Bezugszeichenliste

- 10: Schlauchanordnung
- 11: Einwegschlauch
- 12, 12': Längsachse
- 14: distales Ende
- 16: proximales Ende
- 18: Öffnung
- 20: Austrittsöffnung
- 22: Arbeitskanal
- 24, 24': Spülkanal
- 26, 26': Fixiermanschette
- 28: Führungsschlaufe
- 30: Klebeverbindung
- 32: Verbindungsschlauch
- 34: Innenschlauch
- 36: Abschlusskappe
- 36a, 36b: Abschlusselement
- 38: Verbindungshülse
- 40: Nut
- 42: Kanal
- 44: Endoskop
- 46: Verbindungssteg
- 48: Spiralfeder
- 50: Band
- 52: Taillierung
- 53: Steg
- 54, 54': Schlaufe

- AA, BB, CC: Schnittlinien
- D, E: Verbindung zwischen dem Spülkanal bzw. dem Arbeitskanal und der Umgebung
- FF: Verbindungslinie

## Patentansprüche

1. Schlauchanordnung für ein Endoskop, zumindest mit einem Einwegschlauch (11), der einen Arbeitskanal (22) umgibt, und mit Endoskopbefestigungsmitteln zum Befestigen des Einwegschlauchs (11) an einem Endoskop (44),
wobei die Endoskopbefestigungsmittel eine Fixiereinrichtung (26, 26') aufweisen, durch die der Einwegschlauch (11) am distalen Ende des Endoskops (44) drehfest und axial fest fixierbar ist, und wobei die Endoskopbefestigungsmittel ferner wenigstens eine Führungseinrichtung (28) aufweisen, durch die der Einwegschlauch (11) an einem jeweiligen vom distalen Ende des Endoskops (44) beabstandeten Abschnitt des Endoskops (44) verschieblich befestigbar ist,
**dadurch gekennzeichnet, dass**
die Schlauchanordnung (10) eine Abschlusskappe (36) umfasst, welche mit dem distalen Ende des Einwegschlauchs (11) verbunden ist, wobei die Abschlusskappe (36) fest mit der Fixiereinrichtung (26, 26') verbunden ist.

2. Schlauchanordnung nach Anspruch 1,
**dadurch gekennzeichnet, dass**
die Abschlusskappe (36) einstückig mit der Fixiereinrichtung (26, 26') ausgebildet ist.

3. Schlauchanordnung nach Anspruch 1 oder 2,
**dadurch gekennzeichnet, dass**
die Öffnung (18) des Arbeitskanals (22) und/oder die Austrittsöffnung (20) eines Spülkanals (24) der Schlauchanordnung an der Abschlusskappe (36) ausgebildet sind.

4. Schlauchanordnung nach zumindest einem der vorstehenden Ansprüche,
**dadurch gekennzeichnet, dass**
die Führungseinrichtung (28) mehrere im Wesentlichen hohlzylinderförmig ausgebildete Schlaufen umfasst, die beabstandet voneinander entlang der Länge des Einwegschlauchs (11) angeordnet sind.

5. Schlauchanordnung nach zumindest einem der vorstehenden Ansprüche,
**dadurch gekennzeichnet, dass**
die Führungseinrichtung (28) als ein jeweiliges einstückiges Befestigungselement (50) ausgebildet ist, das den Einwegschlauch (11) und das Endoskop (44) umgibt und das eine Einwegschlauchaufnahme (54) und eine Endoskopaufnahme (54') aufweist, die durch eine Einschnürung (52) und/oder einen Steg (53) definiert sind.

6. Schlauchanordnung nach zumindest einem der vorstehenden Ansprüche,
**dadurch gekennzeichnet, dass**
die Führungseinrichtung (28) und/oder die Fixiereinrichtung (26, 26') fest mit dem Einwegschlauch (11) verbunden oder einstückig mit dem Einwegschlauch (11) ausgebildet ist.

7. Schlauchanordnung nach zumindest einem der vorstehenden Ansprüche,
**dadurch gekennzeichnet, dass**
die Fixiereinrichtung (26, 26') aus elastischem Material besteht und eine axiale Aufnahmeöffnung aufweist, deren Innendurchmesser entlang zumindest eines Teils der Längserstreckung der Fixiereinrichtung für eine reibschlüssige Verbindung mit dem Endoskop (44) dimensioniert ist,
und/oder dass
die Fixiereinrichtung (26, 26') als eine in axialer Richtung beidseitig geöffnete Manschette ausgebildet ist.

8. Schlauchanordnung nach zumindest einem der vorstehenden Ansprüche,
**dadurch gekennzeichnet, dass**
der Einwegschlauch (11) zusätzlich wenigstens einen Spülkanal (24) umgibt, wobei die Schlauchanordnung (10) eine Austrittsöffnung (20) des Spülkanals (24) aufweist, die bezüglich der Fixiereinrichtung (26, 26') für das distale Ende des Endoskops (44) nach distal versetzt angeordnet ist, und wobei die Schlauchanordnung (10) eine Öffnung (18) des Arbeitskanals (22) aufweist, die ebenfalls bezüglich der Fixiereinrichtung (26, 26') für das distale Ende des Endoskops (44) nach distal versetzt angeordnet ist.

9. Schlauchanordnung nach Anspruch 8,
**dadurch gekennzeichnet, dass**
die Austrittsöffnung (20) des Spülkanals (24) als Schlitz ausgebildet ist.

10. Schlauchanordnung nach Anspruch 8 oder 9,
**dadurch gekennzeichnet, dass**
der Spülkanal (24) - bezogen auf einen Querschnitt der Schlauchanordnung (10) - zwischen dem Arbeitskanal (22) und den Endoskopbefestigungsmitteln angeordnet ist.

11. Schlauchanordnung nach einem der Ansprüche 8 bis 10,
**dadurch gekennzeichnet, dass**
die Austrittsöffnung (10) des Spülkanals (24) distal benachbart zu der Fixiereinrichtung (26, 26') für das distale Ende des Endoskops (44) angeordnet ist,
und/oder dass
die Öffnung (18) des Arbeitskanals (22) relativ zu der Austrittsöffnung (20) des Spülkanals (24) nach distal versetzt angeordnet ist.

12. Schlauchanordnung nach zumindest einem der vorstehenden Ansprüche,
**dadurch gekennzeichnet, dass**
die Öffnung (18) des Arbeitskanals (22) und/oder die Austrittsöffnung (20) eines Spülkanals (24) der Schlauchanordnung seitlich angeordnet sind.

13. Schlauchanordnung nach zumindest einem der vorstehenden Ansprüche,
**dadurch gekennzeichnet, dass**
der Arbeitskanal (22) und/oder ein Spülkanal (24) der Schlauchanordnung einen von einer Kreisform abweichenden Querschnitt aufweisen.

14. Schlauchanordnung nach zumindest einem der vorstehenden Ansprüche,
**dadurch gekennzeichnet, dass**
der Einwegschlauch (11) zumindest ein krümmbares Verstärkungselement (48) aufweist, welches insbesondere in einem distalen Bereich der Schlauchanordnung (10) angeordnet ist, der relativ zu der Austrittsöffnung (20) eines Spülkanals (22) der Schlauchanordnung nach proximal versetzt angeordnet ist.

15. Schlauchanordnung nach Anspruch 14,
**dadurch gekennzeichnet, dass**
das Verstärkungselement (48) eine an dem Einwegschlauch (11) befestigte oder in den Einwegschlauch (11) eingebettete Spiralfeder umfasst.

## Claims

1. A hose arrangement for an endoscope, at least having a disposable hose (11) which surrounds a working passage (22) and having endoscope fastening means for the fastening of the disposable hose (11) to an endoscope (44),
wherein the endoscope fastening means have a fixing device (26, 26') by which the disposable hose (11) is rotationally fixedly and axially fixedly fixable to the distal end of the endoscope (44); and
wherein the endoscope fastening means furthermore have at least one guide device (28) by which the disposable hose (11) can be displaceably fastened to a respective section of the endoscope (44) spaced apart from the distal end of the endoscope (44), **characterized in that**
the hose arrangement (10) comprises a closure cap (36) which is connected to the distal end of the disposable hose (11), with the closure cap (36) being fixedly connected to the fixing device (26, 26').

2. A hose arrangement in accordance with claim 1,
**characterized in that**
the closure cap (36) is formed in one piece with the fixing device (26, 26').

3. A hose arrangement in accordance with claim 1 or claim 2,
**characterized in that**
the opening (18) of the working passage (22) and/or the outlet opening (20) of a flushing passage (24) of the hose arrangement is/are formed at the closure cap (36).

4. A hose arrangement in accordance with at least one of the preceding claims,
**characterized in that**
the guide device (28) comprises a plurality of loops which are substantially hollow-cylindrical and which are arranged spaced apart from one another along the length of the disposable hose (11).

5. A hose arrangement in accordance with at least one of the preceding claims,
**characterized in that**
the guide device (28) is formed as a respective one-piece fastening element (50) which surrounds the disposable hose (11) and the endoscope (44) and which has a disposable hose receiver (54) and an endoscope receiver (54') which are defined by a restriction (52) and/or by a web (53).

6. A hose arrangement in accordance with at least one of the preceding claims,
**characterized in that**
the guide device (28) and/or the fixing device (26, 26') is/are fixedly connected to the disposable hose (11) or is/are formed in one piece with the disposable hose (11).

7. A hose arrangement in accordance with at least one of the preceding claims,
**characterized in that**
the fixing device (26, 26') comprises elastic material and has an axial receiver opening whose inner diameter is dimensioned along at least one part of the longitudinal extent of the fixing device for a friction-locking connection to the endoscope (44);
and/or **in that**
the fixing device (26, 26') is formed as a cuff opened at both sides in the axial direction.

8. A hose arrangement in accordance with at least one of the preceding claims,
**characterized in that**
the disposable hose (11) additionally has at least one flushing passage (24), with the hose arrangement (10) having an outlet opening (20) of the flushing passage (24), which is arranged offset to distal with respect to the fixing device (26, 26') for the distal end of the endoscope (44), and with the hose arrangement (10) having an opening (18) of the working passage (22) which is likewise arranged offset to distal with respect to the fixing device (26, 26') for the distal end of the endoscope (44).

9. A hose arrangement in accordance with claim 8,
**characterized in that**
the outlet opening (20) of the flushing passage (24) is formed as a slit.

10. A hose arrangement in accordance with claim 8 or claim 9,
**characterized in that**
the flushing passage (24) is arranged - with respect to a cross-section of the hose arrangement (10) - between the working passage (22) and the endoscope fastening means.

11. A hose arrangement in accordance with any one of the claims 8 to 10,
**characterized in that**
the outlet opening (10) of the flushing passage (24) is arranged distally adjacent to the fixing device (26, 26') for the distal end of the endoscope (44);
and/or **in that**
the opening (18) of the working passage (22) is arranged offset to distal relative to the outlet opening (20) of the flushing passage (24).

12. A hose arrangement in accordance with at least one of the preceding claims,
**characterized in that**
the opening (18) of the working passage (22) and/or the outlet opening (20) of a flushing passage (24) of the hose arrangement is/are arranged laterally.

13. A hose arrangement in accordance with at least one of the preceding claims,
**characterized in that**
the working passage (22) and/or a flushing passage (24) of the hose arrangement has/have a cross-section different from a circular shape.

14. A hose arrangement in accordance with at least one of the preceding claims,
**characterized in that**
the disposable hose (11) has at least one curvable reinforcement element (48) which is in particular arranged in a distal region of the hose arrangement (10) which is arranged offset to proximal relative to the outlet opening (20) of a flushing passage (22) of the hose arrangement.

15. A hose arrangement in accordance with claim 14,
**characterized in that**
the reinforcement element (48) comprises a spiral spring fastened to the disposable hose (11) or embedded in the disposable hose (11).

## Revendications

1. Agencement à tuyau souple pour un endoscope, comprenant au moins un tuyau à jeter (11) qui entoure un canal de travail (22) et comprenant un moyen de fixation d'endoscope pour fixer le tuyau à jeter (11) sur un endoscope (44),
dans lequel le moyen de fixation d'endoscope comprend un dispositif de fixation (26, 26') au moyen duquel le tuyau à jeter (11) est susceptible d'être fixé solidaire en rotation et fixe en direction axiale à l'extrémité distale de l'endoscope (44), et
dans lequel le moyen de fixation d'endoscope comprend en outre au moins un dispositif de guidage (28) au moyen duquel le tuyau à jeter (11) est susceptible d'être fixé de manière déplaçable à une portion respective de l'endoscope (44) à distance de l'extrémité distale de l'endoscope (44),
**caractérisé en ce que**
l'agencement à tuyau souple (10) inclut un capuchon de terminaison (36) qui est relié avec l'extrémité distale du tuyau à jeter (11), et le capuchon de terminaison (36) est relié fermement avec le dispositif de fixation (26, 26').

2. Agencement à tuyau souple selon la revendication 1,
**caractérisé en ce que** le capuchon de terminaison (36) est réalisé d'une seule pièce avec le dispositif de fixation (26, 26').

3. Agencement à tuyau souple selon la revendication 1 ou 2,
**caractérisé en ce que** l'ouverture (18) du canal de travail (22) et/ou l'ouverture de sortie (20) d'un canal de rinçage (24) de l'agencement à tuyau souple sont réalisées sur le capuchon de terminaison (36).

4. Agencement à tuyau souple selon l'une au moins des revendications précédentes,
**caractérisé en ce que** le dispositif de guidage (28) inclut plusieurs boucles réalisées essentiellement en forme de cylindre creux, qui sont agencées à distance les unes des autres le long de la longueur du tuyau à jeter (11).

5. Agencement à tuyau souple selon l'une au moins des revendications précédentes,
**caractérisé en ce que** le dispositif de guidage (28) est réalisé sous forme d'un élément de fixation respectif (50) d'une seule pièce, qui entoure le tuyau à jeter (11) et l'endoscope (44) et qui comporte un récepteur pour tuyau à jeter (54) et un récepteur pour endoscope (54), lesquels sont définis par un rétrécissement (52) et/ou par une barrette (53).

6. Agencement à tuyau souple selon l'une au moins des revendications précédentes,
**caractérisé en ce que** le dispositif de guidage (28) et/ou le dispositif de fixation (26, 26') est relié fermement avec le tuyau à jeter (11) ou est réalisé d'une seule pièce avec le tuyau à jeter (11).

7. Agencement à tuyau souple selon l'une au moins des revendications précédentes,
**caractérisé en ce que**
le dispositif de fixation (26, 26') est en matériau élastique et comporte une ouverture de réception axiale dont le diamètre intérieur, le long d'au moins une partie de l'extension longitudinale du dispositif de fixation, est dimensionné pour assurer une liaison à coopération de friction avec l'endoscope (44),
et/ou **en ce que** le dispositif de fixation (26, 26') est réalisé comme une manchette ouverte des deux côtés en direction axiale.

8. Agencement à tuyau souple selon l'une au moins des revendications précédentes,
**caractérisé en ce que** le tuyau à jeter (11) entoure additionnellement au moins un canal de rinçage (24), ledit agencement à tuyau souple (10) comprenant une ouverture de sortie (20) du canal de rinçage (24), laquelle est agencée décalée en direction distale par rapport au dispositif de fixation (26, 26') pour l'extrémité distale de l'endoscope (44), et ledit agencement à tuyau souple (10) comprenant une ouverture (18) du canal de travail (22), laquelle est également agencée décalée en direction distale par rapport au dispositif de fixation (26, 26') pour l'extrémité distale de l'endoscope (44).

9. Agencement à tuyau souple selon la revendication 8,
**caractérisé en ce que** l'ouverture de sortie (20) du canal de rinçage (24) est réalisée sous forme de fente.

10. Agencement à tuyau souple selon la revendication 8 ou 9,
**caractérisé en ce que** le canal de rinçage (24) est agencé, par référence à une section transversale de l'agencement à tuyau souple (10), entre le canal de travail (22) et le moyen de fixation pour endoscope.

11. Agencement à tuyau souple selon l'une des revendications 8 à 10,
**caractérisé en ce que**
l'ouverture de sortie (10) du canal de rinçage (24) est agencée en situation distale au voisinage du dispositif de fixation (26, 26') pour l'extrémité distale de l'endoscope (44),
et/ou **en ce que** l'ouverture (18) du canal de travail (22) est agencée en décalage en direction distale par rapport à l'ouverture de sortie (20) du canal de rinçage (24).

12. Agencement à tuyau souple selon l'une au moins des revendications précédentes,
**caractérisé en ce que** l'ouverture (18) du canal de travail (22) et/ou l'ouverture de sortie (20) d'un canal de rinçage (24) de l'agencement à tuyau souple sont agencées latéralement.

13. Agencement à tuyau souple selon l'une au moins des revendications précédentes,
**caractérisé en ce que** le canal de travail (22) et/ou un canal de rinçage (24) de l'agencement à tuyau souple présentent une section transversale qui diffère d'une forme circulaire.

14. Agencement à tuyau souple selon l'une au moins des revendications précédentes,
**caractérisé en ce que** le tuyau à jeter (11) comprend au moins un élément de renforcement (48) capable d'être cintré, qui est agencé en particulier dans une région distale de l'agencement à tuyau souple (10), qui est agencé en décalage en direction proximale par rapport à l'ouverture de sortie (20) d'un canal de rinçage (22) de l'agencement à tuyau souple.

15. Agencement à tuyau souple selon la revendication 14,
**caractérisé en ce que** l'élément de renforcement (48) inclut un ressort spiralé fixé sur le tuyau à jeter (11) ou noyé dans le tuyau à jeter (11).
